(19) Europäisches Patentamt
European Patent Office
Office européen des brevets

(11) Publication number: 0 509 241 A2

# (12) EUROPEAN PATENT APPLICATION

(21) Application number: **92104288.3**

(22) Date of filing: **12.03.92**

(51) Int. Cl.[5]: **C07C 205/38**, C07C 201/12, C07C 205/43, C07C 205/57

(30) Priority: **15.03.91 US 669707**

(43) Date of publication of application: **21.10.92 Bulletin 92/43**

(84) Designated Contracting States: **AT CH DE FR GB IT LI**

(71) Applicant: **OCCIDENTAL CHEMICAL CORPORATION**
**2801 Long Road**
**Grand Island New York 14072-1244(US)**

(72) Inventor: **Stults, Jeffrey S.**
**1054 Baseline Road**
**Grand Island, New York 14072(US)**
Inventor: **Buchanan, Robert A.**
**2063 Town Hall Terrace**
**Grand Island, New York 14072(US)**

(74) Representative: **Vossius & Partner**
**Siebertstrasse 4 P.O. Box 86 07 67**
**W-8000 München 86(DE)**

## (54) A novel method for ether formation.

(57) A process for the preparation of 1,1'-oxy-bis(3-trifluoromethyl-4-nitrobenzene) which comprises reacting a compound of the formula

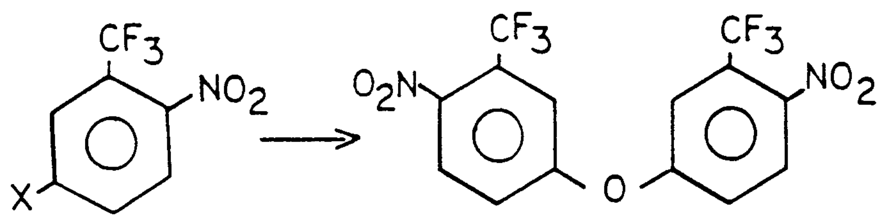

where X is F, Cl, Br or I; with an inorganic base selected from the group consisting of alkali metal hydroxides, alkali metal carbonates, and alkali metal bicarbonates, in the presence of a catalytic quantity of a benzoate catalyst. In addition, benzoate esters of 4-nitro-3-(trifluoromethyl)phenol may be isolated.

EP 0 509 241 A2

This invention relates to a method of forming 1,1'-oxy-bis(3,-trifluoromethyl-4-nitrobenzene) from 5-halo-2-nitrobenzotrifluorides. More particularly, it relates to the use of benzoic acid, and substituted benzoic acids, to catalyze the condensation of two molecules of an aryl halide to yield the diaryl ether.

Aryl ethers have been formed by reacting aryl halides with phenols under various conditions. Roberts and Turner disclose (J. Chem. Soc., 1925, 127, p. 2004) that 2,4-dichloronitrobenzene reacts with phenol in aqueous KOH, at 100°C., to give a quantitative yield of 4-chloro-2-phenoxynitrobenzene.

R. Q. Brewster (Org. Synth. Coll. Vol. 2, p. 445, 1943) teaches the use of a copper catalyst to facilitate the reaction of excess phenol, potassium hydroxide and para-nitrochlorobenzene to form para-nitrophenyl phenyl ether.

J. D. Reinheimer et al, disclose (J. Org. Chem., 1957, vol. 22, p. 1743) that 2,4-dinitrofluorobenzene reacts with 8-hydroxyquinoline in acetone, in the presence of triethylamine, to form 8-(2,4-dinitrophenoxy)-quinoline.

D. J. Brunelle and D. A. Singleton disclose (Tetrahedron Lett., 1984, 25, p. 3383) that the reaction between sodium phenoxide and either para-fluoronitrobenzene or para-chloronitrobenzene, to form para-nitrophenyl phenyl ether may be conducted in chlorobenzene using a phase transfer catalyst. In this case, the phase transfer catalysts studied were n-alkyl salts of 4-dialkylamino pyridines.

Singh and Arora disclose (Tetrahedron 1988, 44, p 2625) that para-chloronitrobenzene dissolved in toluene, may be reacted with solid potassium hydroxide to form 4,4'-dinitrodiphenyl ether. The authors observed the reaction only in the presence of catalytic quantities of various glycols. The authors indicate that the glycols act as phase transfer catalysts. The yields of the ether were poor, that is, never higher than 25%, and there was significant formation of para-chloroaniline. The authors performed mechanistic studies which led them to the conclusion that the reaction proceeded by a free radical chain mechanism.

U. S. Patent 4,558,164 discloses a process for preparing a symmetrical dinitrophenyl ether from either ortho or para-nitrochlorobenzenes or ortho or para-nitrofluorobenzene, comprising using a polar organic solvent, a potassium salt of a fatty carboxylic acid containing 2 to 20 carbon atoms or a potassium salt of an aromatic carboxylic acid containing 7 to 12 carbon atoms as catalyst, and either sodium or potassium carbonate to react with the para-nitrochlorobenzene. The reaction is carried out at from 150° C to 210°C until the ortho or para-nitrochlorobenzene or ortho or para-nitrofluorobenzene reacts. Only aprotic polar solvents are useful and dimethylacetamide is the preferred solvent.

Japanese Patent 61200947 (as abstract in CA 106:156046 and in Derwent Accession number 86-275567/42) discloses a process for the preparation of 3,4'- and 4,4'-dinitrodiphenyl ether. In this process 4-chloronitrobenzene is mixed with a nitrophenol, a hydrogen chloride absorbent such as an alkaline metal hydroxide or bicarbonate, in the presence of 5 to 50 weight percent of the total material of polyethylene glycol of molecular weight 400 to 1000 or its lower alkyl ethers. The entire mixture is heated to a temperature being between 100 and 240°C. There is no additional solvent material used.

Maki and Inukai (Gov. Ind. Res. Inst. Nagoya, Nagoya, Japan). Nippon Kagaku Kaishi (3), 675-7 (Japan) 1972, condensed chloronitrobenzotrifluorides with alkali metal carbonates to produce bis-trifluoromethyl-dinitrophenyl ethers. Specifically, they used 2-chloro-5-nitrobenzotrifluoride to produce the corresponding trifluoromethyl nitrodiphenyl ether. They also studied 5-chloro-2-nitrobenzotrifluoride. In order to accomplish the condensation, they used alkali metal carbonates in 1,3-dimethyl-2-imidazolidinone (DMI). No yields or other experimental details were reported.

In our own laboratories we found that the simple reaction of 2-chloro-5-nitrobenzotrifluoride with potassium carbonate led to side reactions. Various reactions were conducted in polar solvents such as DMAC (at 140-150°C) or non-polar solvents, or even neat in the presence of phase transfer catalysts (18-crown-6, tetraphenyl phosphonium bromide, or the methyl ethers of polyethylene glycols) at temperatures over 200°C. Unfortunately, an appreciable quantity of products such as 2-fluoro-5-nitrobenzotrifluoride were formed. Since there is no other source of fluorine, the appearance of these products indicated that there was some decomposition of the trifluoromethyl group.

Jacobs discloses (Journal of Organic Chemistry v. 36; p. 242; 1971) that when 4-nitro-3-(trifluoromethyl)-chlorobenzene (this molecule may also be named 5-chloro-2-nitrobenzotrifluoride) is treated with sodium hydroxide in dimethyl sulfoxide (DMSO), the trifluoromethyl group is completely removed and the product is 5-chloro-2-nitrophenol which is isolated in 93% yield.

Chaw, Fischer, and Happer, J. Chem. Soc. (B) 9, 1918 - 19 (1971), disclose the synthesis of the benzoate ester of 4-nitro-3-(trifluoromethyl)-phenol ($\alpha,\alpha,\alpha$-trifluoro-m-cresol benzoate by the reaction of $\alpha,\alpha,\alpha$-trifluoro-m-cresol with benzoyl chloride in pyridine.

It has now been found that substituted benzoic acids, and substituted benzoates, when present in catalytic quantities, catalyze the reaction of 5-halo-2-nitrobenzotrifluoride with an inorganic base and water to form 1,1'-oxy-bis(3-trifluoromethyl-4-nitrobenzene). In addition, if benzoate is present in sufficient quantity, benzoate esters of 4-nitro-3-(trifluoromethyl)phenol are formed. The reaction may be run in the absence of a solvent, in a dipolar aprotic solvent, or in a non-polar solvent.

Surprisingly, it has now been found that benzoate catalysts, that is, benzoic acid and its salts and substituted benzoic acids, and their salts, with electron withdrawing substituents, when present in catalytic quantities, catalyze the reaction of 5-halo-2-nitro-benzotrifluoride and an inorganic base such as sodium carbonate, to form 1,1'-oxy-bis(3-trifluoromethyl-4-nitrobenzene). As set forth more fully below, the substituted benzoic acids may have either electron donating or electron withdrawing substituents. In addition, if benzoate is present in sufficient quantity, benzoate esters of 4-nitro-3-(trifluoromethyl)phenol are formed by the displacement of the halide by the benzoate anion. The halogen in the starting material may be F, Cl, Br or I. The starting material may be prepared by the nitration of a 3-halobenzotrifluoride. The chloro compound, that is 5-chloro-2-nitrobenzotrifluoride, is commercially available.

The reaction is run between about 110 ° C and 220 ° C. The overall equation for the for the formation of the ether is:

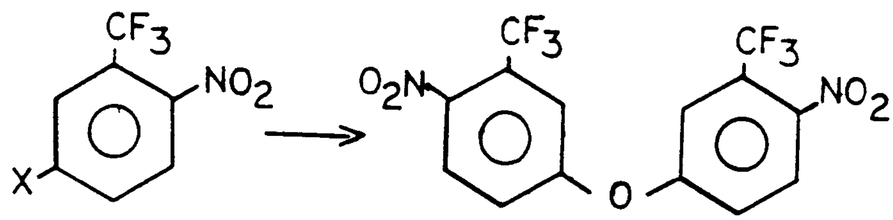

and the overall equation for the formation of esters is

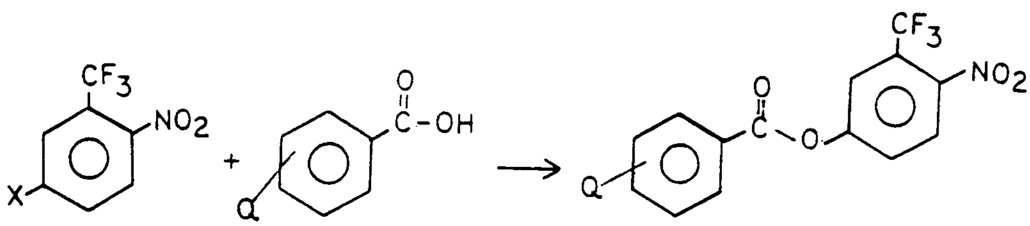

where X is F, Cl, Br, or I.

The reaction may be conducted in a non polar solvent with a boiling point of greater than about 150 ° C. Alternatively the reaction may be run in a non-polar solvent of lower boiling point provided that it is run under sufficient pressure to achieve the temperatures necessary for the reaction. Among the non-polar solvents which may be used are aromatic hydrocarbons such as benzene, toluene, xylene, and chlorinated aromatic hydrocarbons such as trichlorobenzene, dichlorobenzene and $\alpha$-chloronaphthalene.

When the reaction is run at a non-polar solvent, a phase transfer catalyst, such as the methyl ether of polyethylene glycols, crown ethers, phosphonium salts, or ammonium salts are required. Generally speaking, a phase transfer catalyst is a chemical species which combines, within the same molecule both a polar and a non-polar end. Such molecules allow polar species to exist in non-polar solvents. The reaction may be conducted in the absence of a solvent using a phase transfer catalyst and conditions similar to those used for a non-polar solvent.

The reaction may be run in a dipolar aprotic solvent. In this case, no phase transfer catalyst are required. Among the solvent which are useful are dimethyl acetamide, dimethyl formamide, N-methyl pyrrolidone, dimethyl sulfoxide, and sulfolane.

Whatever solvent is chosen for the reaction, a small amount of water is required for the reaction to proceed. However, water generally need not be added to the reaction since either the base, or the ingredients themselves, generally contain sufficient water for the reaction to occur. Accordingly, the addition of extra water is hardly ever necessary. Most dipolar aprotic solvents contain more water than is required for the reaction.

Alkali metal hydroxides, carbonates, and bicarbonates are suitable bases for conducting this reaction. The carbonates are the preferred bases. Although potassium carbonate and sodium carbonate are usable, cost considerations make sodium carbonate the preferred base. The choice of which benzoate to use is influenced by the base chosen for the reaction. For example, if carbonate is chosen as the base, then the

reaction is best catalyzed by benzoates with moderately electron withdrawing constituents such as chlorine. On the other hand, when hydroxides are chosen as the base, electron rich benzoates, such as 4-methoxybenzoic acid, are useful catalysts. In general, weaker bases work better with benzoates containing electron withdrawing substituents, while stronger bases work better with benzoates containing electron donating substituents. Whatever base is selected, it is important that a stoichiometric amount of base, or less, be used. Larger amounts of base may lead to side reactions, and, accordingly, are undesirable. It may also be desirable to add the base incrementally rather than in one portion in order to avoid side reactions. Any benzoate catalyst which is stable under the reaction conditions may be used as a catalyst for this reaction. For example, chlorobenzoic acids, bromobenzoic acids, fluorobenzoic acids, dichlorobenzoic acids, dibromobenzoic acids, tribromobenzoic acids, trichlorobenzoic acids, trifluoromethylbenzoic acids, nitrobenzoic acids, dinitrobenzoic acids, methylbenzoic, and methoxybenzoic acids or salts of any of the above acids may be used as catalysts for this reaction. The catalyst is effective in amounts of about 0.1 mole % or more.

The catalytic effect of the benzoate is illustrated by comparing a reaction run without benzoate, with a similar reaction run in the presence of a benzoate. This can be readily seen by examining the results shown in Comparative Example 1, compared to the results shown by Examples 1 - 4. The comparative example, run without benzoate catalyst, shows much poorer yields and slower conversion than is shown by the same reaction run with benzoate. For example, in Example 2, after 11 hours of reaction there was 86.8% conversion whereas in Comparative Example 1 after the same time there was only 6.5% conversion. Similarly, Comparative Example 2 may be compared to Example 5. In Example 5, after 3.5 hours, there was 62% conversion and, in the absence of catalyst in Comparative Example 2, there was 0.4% conversion. Comparative Example 3 may be compared with Example 6. After 5 hours, in Example 6, GC analysis indicated that the yield of the desired product would be 1.8 g. In Comparative Example 3, using the same amount of reactant and the same reaction time without the presence of catalyst, GC analysis indicated that the yield of the desired product would 0.03 g. Comparative Example 4 may be compared to Example 7. In Example 7, after 1.75 hours, the GC analysis indicated that the yield of the desired product would be 4 g. In Comparative Example 4, in the absence of catalyst, using the same quantity of reactants, the GC analysis indicated that the yield of the desired product would be 2.9 g.

Formation of the ester can be increased by conducting the reaction in the presence of a minimal amount of water, by limiting the amount of base, or by using the salt of the benzoic acid as a reagent. Yields of the benzoyloxy esters can be maximized and the amount of the ether minimized by using a stoichiometric amount of the benzoic acid salt in the absence of additional base.

The product of this reaction may be isolated by filtering the reaction mixture to remove inorganic matter and evaporating the solvent. The resulting product may be crystallized from an alcohol such as methanol or ethanol. Other methods of isolation and purification will be apparent to those skilled in the art.

## EXAMPLE 1

5-chloro-2-nitrobenzotrifluoride (2.26 g), potassium carbonate (0.89 g), and 4-anisic acid (17.1 mg) were heated in DMF (5 mL) at 145° C. The reaction was monitored by GC and the results are given in the table below.

| Time (hrs) | CBTFNB* | OBTFNB** | Conversion |
|---|---|---|---|
| 1.0 | 81.0 | 11.6 | 13% |
| 2.6 | 71.5 | 19.4 | 21.3% |
| 11.0 | 27.7 | 48.4 | 63.6% |

*CBTFNB = 5-chloro-2-nitrobenzotrifluoride;
**OBTFNB = 1,1'-Oxy-bis(3-trifluoromethyl-4-nitrobenzene)

## EXAMPLE 2

5-chloro-2-nitrobenzotrifluoride (2.26 g), potassium carbonate (0.89 g), and 4-chlorobenzoic acid (17.3 mg) were heated in DMF (5 mL) at 145° C. The reaction was monitored by GC and the results are given in the table below.

| Time (hrs) | CBTFNB | OBTFNB | Conversion |
|---|---|---|---|
| 1.0 | 71.1 | 21.8 | 23.5% |
| 2.6 | 57.2 | 33.5 | 36.9% |
| 11.0 | 10.5 | 68.8 | 86.8% |

**EXAMPLE 3**

5-chloro-2-nitrobenzotrifluoride (2.26 g), potassium carbonate (0.89 g), and 4-trifluoromethylbenzoic acid (19 mg) were heated in DMF (5 mL) at 145° C. The reaction was monitored by GC and the results are given in the table below.

| Time (hrs) | CBTFNB | OBTFNB | Conversion |
|---|---|---|---|
| 1.0 | 85.6 | 6.95 | 7.5% |
| 2.6 | 76.5 | 14.0 | 15.5% |
| 11.0 | 38.1 | 42.5 | 52.7% |

**EXAMPLE 4**

5-chloro-2-nitrobenzotrifluoride (22.6 g, 90% pure), potassium carbonate (9.5 g), and 4-chlorobenzoic acid (0.27 g) were heated in DMF (50 mL) at 145° C for 11 hours. The DMF was distilled at reduced pressure and toluene (80 mL) was added. The mixture was heated and the toluene layer was decanted and allowed to stand overnight. The solid was collected, washed, and dried to give 5.8 g 1,1'-oxy-bis(3-trifluoromethyl-4-nitrobenzene). The residue from the toluene decantation was dissolved in ethyl acetate/water (50 mL each). The ethyl acetate layer was separated from the water, dried, and combined with the toluene mother liquor. The combined organic layer was distilled to remove ethyl acetate and about 50 mL. of toluene. The distillation pot was allowed to cool and more 1,1'-oxy-bis(3-trifluoromethyl-4-nitrobenzene) precipitated (2.4 g).

**EXAMPLE 5** 1,1'-Oxy-bis(3-trifluoromethyl-4-nitrobenzene)

5-chloro-2-nitrobenzotrifluoride (4.6 g, 20 mmol), potassium 4-chlorobenzoate (0.18 g, 1.0 mmol, 5 mole%), 18-crown-6 (0.19 g, 0.71 mmol), and potassium carbonate (1.34 g, 10 mmol) were heated to 130 - 135° C. The reaction was monitored by GC area percent with the results shown below.

| GC Area Percent | | | |
|---|---|---|---|
| Time | CNBTF | OBTNB | Conversion |
| 0.5 hrs | 86.5 | 8.20 | 8.7% |
| 2.0 hrs | 60.3 | 31.7 | 35% |
| 3.5 hrs | 33.9 | 54.9 | 62% |

The material was dissolved in refluxing toluene (15 mL) and filtered to remove salts. The solution was allowed to cool to room temperature and the resulting solid was collected, washed, and dried to give 1,1'-oxy-bis(3-trifluoromethyl-4-nitrobenzene) as a solid (2.0 g). GS-ISTD analysis of the mother liquor indicated an additional 0.7 g of product was present for a total yield of 2.7 g (67% yield, >95% yield based on conversion). Compare to Comparative Example 2.

**EXAMPLE 6** 1,1'-Oxy-bis(3-trifluoromethyl-4-nitrobenzene)

5-Chloro-2-nitrobenzotrifluoride (4.6 g, 20 mmol), sodium 3-nitrobenzoate, hemihydrate (0.20 g, 1.0 mmol, 5 mole%), 18-crown-6 (0.19 g, 0.71 mmol), potassium carbonate (1.33 g, 10 mmol), and 1,2,4-trichlorobenzene (4.4 g, 3.0 mL) were heated to 145-150° C for 5 hours. The reaction was monitored by GC

area percent with the results shown below.

| GC Area Percent | | | | |
|---|---|---|---|---|
| Time | CNBTF | TCB | OBTNB | Conversion |
| 0.5 hrs | 50.7 | 4.9 | 1.49 | 2.9% |
| 1.5 hrs | 46.5 | 43.5 | 6.28 | 11.9% |

The material was dissolved, after cooling, in methylene chloride (25 mL) and filtered to remove salts. The solution was analyzed by GC-ISTD which showed the presence of 1,1'-oxy-bis(3-trifluoromethyl-4-nitrobenzene) (1.8 g). Compare to Comparative Example 4.

## EXAMPLE 7

5-Chloro-2-nitrobenzotrifluoride (15.5 g, 60 mmol) and potassium 4-anisate (0.45 g, 2.4 mmol, 4% catalyst loading) were heated in DMSO (30 mL) 145-150 °C. Sodium hydroxide (Occidental Chemical, beads, Diamond grade, 2.36 g, 59 mmol) was added beadwise over 1.25 hours. The reaction was heated for an additional 30 minutes and sampled. GC-ISTD analysis of the reaction mixture indicated the presence of 1,1'-oxy-bis(3-trifluoromethyl-4-nitrobenzene) (4.04 g). Compare to Comparative Example 4.

## COMPARATIVE EXAMPLE 1

5-chloro-2-nitrobenzotrifluoride (2.26 g) and potassium carbonate (0.9 g) were heated in DMF (5 mL) at 145 °C. The reaction was monitored by GC and the results are given in the table below.

| Time (hrs) | CBTFNB | OBTFNB | Conversion |
|---|---|---|---|
| 1.0 | 93.1 | 0 | 0% |
| 2.6 | 91.2 | 0.5 | 0.5% |
| 11.0 | 81.0 | 5.6 | 6.5% |

## COMPARATIVE EXAMPLE 2

1,1'-Oxy-bis(3-trifluoromethyl-4-nitrobenzene)

5-Chloro-2-nitrobenzotrifluoride (4.6 g, 20 mmol), 18-crown-6 (0.19 g, 0.71 mmol), and potassium carbonate (1.34 g, 10 mmol) were heated to 130-135 °C. The reaction was monitored by GC area percent with the results shown below.

| GC Area Percent | | | |
|---|---|---|---|
| Time | CNBTF | OBTNB | Conversion |
| 0.5 hrs | 94.7 | 0.18 | 0.2% |
| 2.0 hrs | 93.6 | 0.40 | 0.4% |
| 3.5 hrs | 91.1 | 0.40 | 0.4% |

Compare to Example 5.

## COMPARATIVE EXAMPLE 3

5-Chloro-2-nitrobenzotrifluoride (4.6 g, 20 mmol), 18-crown-6 (0.19 g, 0.71 mmol), potassium carbonate (1.33 g, 10 mmol), and 1,2,4-trichlorobenzene (4.4 g, 3.0 mL) were heated to 145-150 °C for 5 hours. The reaction was monitored by GC area percent with the results shown below.

| GC Area Percent | | | | |
|---|---|---|---|---|
| Time | CNBTF | TCB | OBTNB | Conversion |
| 0.5 hrs | 53.1 | 44.3 | 0.05 | 0.1% |
| 1.5 hrs | 53.3 | 44.2 | 0.12 | 0.2% |

The material was dissolved, after cooling, in methylene chloride (25 mL) and filtered to remove salts. The solution was analyzed by GC-ISTD which showed the presence of 1,1'-oxy-bis(3-trifluoromethyl-4-nitrobenzene) (0.03 g). Compare to Example 6

**COMPARATIVE EXAMPLE 4**

5-Chloro-2-nitrobenzotrifluoride (15.5 g, 60 mmol) was heated in DMSO (30 mL) to 145-150°C. Sodium hydroxide (Occidental Chemical, beads, Diamond grade, 2.36 g, 59 mmol) was added beadwise over 1.25 hours. The reaction was heated for an additional 30 minutes and sampled. GC-ISTD analysis of the reaction mixture indicated the presence of 1,1'-oxy-bis(3-trifluoromethyl-4-nitrobenzene) (2.9 g). Compare to Example 7.

## Claims

1. A process for the preparation of 1,1'-oxy-bis(3-trifluoromethyl-4-nitrobenzene) which comprises reacting a compound of the formula

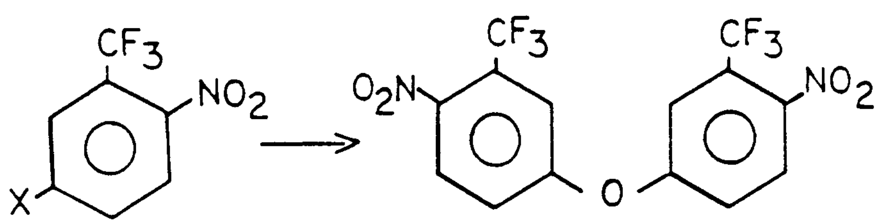

where X if F, Cl, Br or I; with an inorganic base selected from the group consisting of alkali metal hydroxides, alkali metal carbonates, and alkali metal bicarbonates, in the presence of a catalytic quantity of a benzoate catalyst.

2. A process according to Claim 1 conducted in a solvent

3. A process according to Claim 2 conducted in a non-polar solvent in the presence of a phase transfer catalyst,

4. A process according to Claim 2 conducted in a polar aprotic solvent

5. A process according to any one of claims 1 to 4 wherein the inorganic base is sodium carbonate or potassium carbonate.

6. A process according to any one of claims 1 to 4 wherein the inorganic base is sodium hydroxide or potassium hydroxide.

7. A compound of the formula

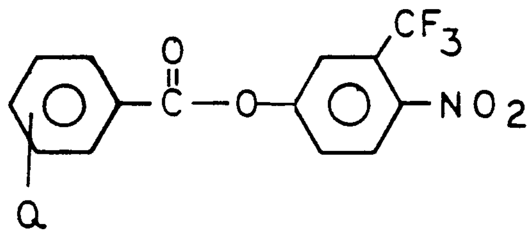

where Q is selected from the group consisting of Cl, F, Br, $NO_2$, 3,4-diCl, 3,4-diBr, 3,4-diF, 3,4-di$NO_2$, $CF_3$, $OCH_3$, $CH_3$, H, and $CO_2R$ where R is selected from the group consisting of H or a C-1 to C-12 alkyl group.

**8.** A compound according to Claim 11 where Q = 3-Cl, 4-Cl, H, 3-$NO_2$, 4-$NO_2$, or 4-$OCH_3$.